# EUROPEAN PATENT SPECIFICATION

(11) **EP 4 389 191 B1**
(45) Date of publication and mention of the grant of the patent: **08.07.2026**
(21) Application number: 22214571.6
(22) Date of filing: 19.12.2022
(51) Int. Cl.: A61N 1/04, A61N 1/05, A61N 1/32, A61N 1/36

(54) **A DEVICE FOR PROVIDING BRAIN STIMULATION AND/OR NERVE STIMULATION**
VORRICHTUNG ZUR BEREITSTELLUNG VON HIRNSTIMULATION UND/ODER NERVENSTIMULATION
DISPOSITIF POUR FOURNIR UNE STIMULATION CÉRÉBRALE ET/OU UNE STIMULATION NERVEUSE

(43) Date of publication of application: 26.06.2024
(73) Proprietor: Stichting IMEC Nederland, 5656 AE Eindhoven (NL)
(72) Inventor: ROSSETTI, Nicolo, 5654 AN Eindhoven (NL); MIHAJLOVIC, Vojkan, 5643 AZ Eindhoven (NL); KOSUNEN, Ilkka, 3581 EE Utrecht (NL)
(74) Representative: AWA Sweden AB

(56) References cited:
- US-A1- 2019 366 087
- US-A1- 2020 324 119
- US-A1- 2022 362 554

## Description

### Technical field

The present description relates to stimulation of a brain and/or a nerve of a living being. In particular, the present description relates to a device for providing brain stimulation and/or nerve stimulation and presents a method for providing brain stimulation and/or nerve stimulation in which the device may be used.

### Background

Neurostimulation may be used for controlling a function of an organ or part of a body of a living being, such as a human being, e.g., for alleviating or treating a number of conditions of the human being. Neurostimulation may involve a control of electrical signals transported through a brain and/or a nerve, such as a part of peripheral nervous system.

Neurostimulation may address stimulating temporal dynamics of neural signals. Thus, the neurostimulation may be configured to stimulate a periodical, cyclic pattern of neural signals, such as brain waves propagating in a brain or a periodical occurrence of a signal through a nerve.

Neurostimulation may be achieved by interferential stimulation, such as temporal interference stimulation (TIS). In such case, temporal interference of two stimulation signals may be used, wherein interference of the two stimulation signals generates an interferential signal that may cause stimulation of the brain and/or nerve. Interferential stimulation may allow selectively targeting regions within the brain and/or nerve such that stimulation occurs in regions extending (deep) into the neural tissue, while the stimulation signals may be emitted from a location external to the neural tissue.

However, efficiency of the interferential stimulation may depend on a relation between the interferential signal and neural signal propagating in the brain and/or nerve. Therefore, it is desired to improve control of interferential stimulation in relation to neural signal propagating in the brain and/or nerve.

US 2019/366087 A1 discloses an interferential current system for performing a therapeutic procedure includes a controller, a stimulation power supply and at least one sensor providing patient derived sensor feedback to the controller. The system also includes at least two electrodes disposed on an epidermis of the patient and arranged to supply transcutaneous electrical impulses to a therapeutic target area when supplied power by the stimulation power supply. The electrodes supply impulses at two different frequencies, giving rise to at least one beat impulse having an interference frequency.

US 2020/324119 A1 discloses a system for delivering neurostimulation energy including a programming control circuit and a user interface. The programming control circuit may be configured to generate stimulation parameters according to a neurostimulation program including a pattern of interferential stimulation configured to effect asynchronous and/or non-regular activation of nerve fibers by simultaneously delivering a first stimulation current having a first waveform with a first frequency using a first electrode configuration and a second stimulation current having a second waveform with a second frequency using a second electrode configuration.

### Summary

An objective of the present description is to provide accurate control of stimulation of a brain and/or a nerve in relation to neural signals.

This and other objectives are at least partly met by the invention as defined in the independent claim 1. Preferred embodiments are set out in the dependent claims.

According to a first aspect, the present description relates to a device for providing brain stimulation and/or nerve stimulation of a living being, said device comprising: a stimulation generating unit configured to generate at least a first high frequency stimulation signal and a second high frequency stimulation signal for stimulating a brain and/or a nerve of the living being, wherein a difference between a first frequency of the first high frequency stimulation signal and a second frequency of the second high frequency stimulation signal defines a beat frequency; a set of electrodes configured to receive the first high frequency stimulation signal and the second high frequency stimulation signal and configured to be arranged in relation to the brain and/or the nerve for transmitting the first high frequency stimulation signal and the second high frequency stimulation signal into the brain and/or the nerve, wherein the set of electrodes are configured to cause interferential stimulation in the brain and/or the nerve by an interferential stimulation signal having the beat frequency, wherein the interferential stimulation signal is formed through interference by the first high frequency stimulation signal and the second high frequency stimulation signal; a measurement unit, comprising at least two sensing electrodes, wherein the measurement unit is configured to acquire a measurement of at least a neural signal propagating in the brain and/or the nerve; a processing unit, configured to receive the measurement from the measurement unit and configured to determine a phase relation between the neural signal and the interferential stimulation signal and configured to control brain stimulation and/or nerve stimulation based on the determined phase relation.

The device according to the first aspect uses a first and a second high frequency stimulation signal. The brain and/or nerve may be affected to a larger extent by low frequency signals. This implies that the frequency of the first and the second stimulation signals, respectively, may be sufficiently high so as not to affect or insignificantly affect neural tissue. Further, the first and the second high frequency stimulation signal are used to generate an interferential stimulation signal that will exhibit a beat frequency based on a difference between the first frequency and the second frequency. The beat frequency may have a low frequency lower than the first frequency and the second frequency. The beat frequency may be adapted to provide stimulation of the brain and/or nerve.

Further, thanks to the interference between the first and second stimulation signals, an amplitude of the first high frequency stimulation signal and an amplitude of the second high frequency stimulation signal may be smaller than an amplitude of the interferential signal with the beat frequency at a location in the brain and/or nerve in which an effect on the brain and/or nerve is desired. Thus, a sufficiently large signal (amplitude) for stimulating the brain and/or nerve may be provided only by the interferential signal. Hence, the stimulation of the brain and/or nerve may be solely provided by the interferential signal.

This implies that the device may be able to control a location (deep) within the brain and/or nerve, where stimulation is to be provided, without the device providing stimulation in undesired locations of the brain and/or nerve.

The device is further configured to determine a phase relation between the neural signal and the interferential stimulation signal such that brain stimulation and/or nerve stimulation can be controlled based on the determined phase relation. This implies that the device may ensure that the stimulation is provided with a desired phase relation to a neural signal.

For instance, brain stimulation may be provided in phase or out of phase with a brain wave so as to strengthen a desired brain wave or suppress an undesired brain wave. Also, nerve stimulation may be provided to strengthen or suppress neural signals propagating in the nerve.

It should be realized that stimulation of the brain and/or the nerve may imply that neural signals are strengthened or suppressed. The interferential stimulation signal may thus cause an effect on the brain and/or the nerve which does not necessarily mean that a neural signal is transmitted in the brain and/or nerve but may alternatively mean that a neural signal is prevented from being transmitted in the brain and/or nerve.

The first and the second frequency may be dependent on a type of stimulation to be used. For instance, for brain stimulation, the first and the second frequency may be larger than 60 Hz, such as in a range of 60 - 100 Hz, or larger than 100 Hz, such as in a range of 150 - 250 Hz or even extending into a range of kHz or MHz frequencies. Such frequencies may differentiate the high frequency stimulation signals from normal frequency content of the neural activity in the brain. However, it should be realized that in some situations, a subject may exhibit higher frequency oscillations in the brain activity, such as in case of epileptic seizures. Thus, the first and the second frequency may be at least 1 kHz or even larger. For nerve stimulation, the first and the second frequency may typically be larger than for brain stimulation. For instance, the first and the second frequency may be in a range of 500 Hz - 1 MHz, such as 10 - 30 kHz.

The first and the second frequency may preferably be sufficiently high so as not to affect the nerve. The first and the second frequency may preferably not be too high so that a relatively small difference in the frequencies may still be accurately defined.

The beat frequency used may depend on the effect on the brain and/or nerve that is desired. For brain stimulation, the beat frequency may correspond to frequencies of brain waves such that the beat frequency may be in a range of 0.5 - 45 Hz, such as 8 - 12 Hz. For nerve stimulation, the beat frequency may correspond to a repetition frequency of neural signals in the nerve, such as beat frequencies in a range of 5 Hz - 2 kHz.

The set of electrodes may comprise a first pair of electrodes to be used for transmitting the first high frequency stimulation signal and a second pair of electrodes to be used for transmitting the second high frequency stimulation signal. The set of electrodes may further comprise more electrodes allowing further pairs of electrodes to be used for transmitting further stimulation signals that may contribute to the forming of the interferential stimulation signal.

The pairs of electrodes may be statically defined in the set of electrodes. However, it should be realized that the electrodes to be used for transmitting stimulation signals may be dynamically selected. This implies that a location of a pair of electrodes in relation to the brain and/or nerve may be selected when a stimulation signal is generated.

According to an embodiment, the neural signal exhibits a neural frequency and wherein the processing unit is configured to determine the phase relation at the neural frequency.

The phase relation may thus provide information of the relation between the neural signal and the interferential stimulation signal at the neural frequency. The processing unit may thus further control brain stimulation and/or nerve stimulation to ensure that a desired phase relation between the neural signal and the interferential stimulation signal is provided. This may imply that the interferential stimulation signal may be controlled to, for instance, be in phase or out of phase with the neural signal for strengthening the neural signal or suppressing the neural signal.

The neural frequency may be a frequency defining a varying amplitude of the neural signal. The neural frequency may alternatively be a repetition frequency of pulses of the neural signal.

According to an embodiment, the stimulation generating unit is configured to generate the first high frequency stimulation signal and the second high frequency stimulation signal for defining the beat frequency to equal the neural frequency.

Thus, the interferential stimulation signal may have a beat frequency corresponding to the neural frequency such that a time variation of the interferential stimulation signal corresponds to characteristics of the neural signal. This implies that the interferential stimulation signal may be configured to strengthen or suppress the neural signal, e.g., strengthen or suppress an amplitude of the neural signal, based on electrochemical effects taking place at neurons (i.e., nerve fibers) resulting from the interferential stimulation signal.

According to an embodiment, the measurement unit is configured to acquire a measurement of the interferential stimulation signal and the neural signal, wherein the processing unit is configured to determine the phase relation based on analysis of the neural frequency and at least one of the first frequency of the first high frequency stimulation signal or the second frequency of the second high frequency stimulation signal.

The measurement by the measurement unit may thus comprise information relating to both the interferential stimulation signal and the neural signal. The phase relation may thus be extracted based on a measurement that provides information of both the interferential stimulation signal and the neural signal. This facilitates determination of the phase relation.

According to an embodiment, the processing unit is configured to determine phase information of the interferential stimulation signal based on analysis of at least one of the first frequency of the first high frequency stimulation signal or the second frequency of the second high frequency stimulation signal.

Hence, the determination of the phase relation may use at least one of the first frequency and the second frequency, which do not correspond to the beat frequency (or the neural frequency). This may facilitate extraction of phase information of the interferential stimulation signal as analysis may be made of a frequency which does not correspond to the beat frequency.

The phase information may be determined through envelope extraction at the at least one of the first frequency and the second frequency. For instance, the determination of the phase information may use wavelet analysis.

According to an embodiment, the stimulation generating unit is configured to generate a sequence of bursts of the first high frequency stimulation signal and the second high frequency stimulation signal defining a repetition frequency of the bursts in the sequence, wherein the processing unit is configured to control a phase relation such that onset of a burst of the interferential stimulation signal is in desired phase relation with the neural signal at the neural frequency.

Thus, the interferential stimulation signal may provide a sequence of bursts. The bursts of the interferential stimulation signal may be controlled in relation to the neural signal. This may be used for providing bursts at corresponding time instances with neural signals propagating in the brain and/or nerve. For instance, a phase angle of the interferential stimulation signal may be controlled for achieving a desired effect.

According to an embodiment, the stimulation generating unit is configured to generate the first high frequency stimulation signal and the second high frequency stimulation signal during a period of time and wherein the measurement unit is configured to acquire the measurement during the period of time and the processing unit is configured to determine the phase relation during the period of time.

Thus, the interferential stimulation signal may provide stimulation of the brain and/or nerve while a phase relation is determined. Hence, the stimulation may be controlled based on the determined phase relation while stimulation is provided.

According to an embodiment, the processing unit is configured to control the stimulation generating unit to adapt at least one of the first high frequency stimulation signal or the second high frequency stimulation signal in order to change the phase relation between the neural signal and the interferential stimulation signal.

Thus, the phase relation may be changed by adapting one of the first and the second high frequency stimulation signal. This implies that the phase relation may also be changed continuously while interferential stimulation is provided to the brain and/or nerve, by the phase relation being measured and updated while stimulation is provided.

According to an embodiment, the stimulation generating unit is configured to generate a third high frequency stimulation signal and a fourth high frequency stimulation signal for stimulating a brain and/or a nerve of the living being, wherein a difference between a third frequency of the third high frequency stimulation signal and a fourth frequency of the fourth high frequency stimulation signal defines a beat frequency; wherein the set of electrodes comprises a first sub-set of electrodes configured to receive the first high frequency stimulation signal and the second high frequency stimulation signal and configured to be arranged at a first location in relation to the brain and/or the nerve for causing interferential stimulation by a first interferential stimulation signal forming said interferential signal, and wherein the device further comprises a second sub-set of electrodes configured to receive the third high frequency stimulation signal and the fourth stimulation signal and configured to be arranged at a second location different from the first location in relation to the brain and/or the nerve for transmitting the third high frequency stimulation signal and the fourth high frequency stimulation signal into the brain and/or the nerve, wherein the second set of electrodes are configured to cause interferential stimulation in the brain and/or the nerve by a second interferential stimulation signal having the beat frequency, wherein the second interferential stimulation signal is formed through interference by the third high frequency stimulation signal and the fourth high frequency stimulation signal; wherein the processing unit is configured to determine a phase relation between the neural signal and the first interferential stimulation signal and wherein the processing unit is further configured to control the second interferential stimulation signal based on the determined phase relation.

Hence, the processing unit may be configured to acquire a measurement of a neural signal in combination with a first interferential stimulation signal. The processing unit may further be configured to determine a phase relation between the neural signal and the second interferential stimulation signal so as to control the second interferential stimulation signal in relation to the neural signal. For instance, the measurement of the first interferential stimulation signal may be used for determining a conduction velocity of the neural signal which may be used for controlling the phase relation of the second interferential stimulation signal, e.g., the phase angle of the second interferential stimulation signal, such that the second interferential stimulation signal is provided in a desired relation to the neural signal propagating in the brain and/or nerve.

According to an embodiment, the processing unit is configured to control the second interferential stimulation signal for controlling blocking or strengthening a neural signal triggered by the first interferential stimulation signal.

The device may thus be used for providing an increased control of stimulation of the brain and/or nerve by providing a combined effect of the stimulation by the first interferential stimulation signal and the second interferential stimulation signal.

The first interferential stimulation signal may trigger generation of a neural signal in a nerve that may be configured to transmit signals to / from various parts of the body. For instance, the vagus nerve is a cranial nerve providing signals between the brain and parts of the body. The vagus nerve may be relatively easy to access and a device for stimulating a nerve that is to be implanted in the human being may therefore be advantageously arranged in relation to the vagus nerve for providing stimulation of the vagus nerve. However, since the vagus nerve provides signals to many different parts of the body, it is also important that stimulation of the nerve is controlled to provide a desired effect of the stimulation. The first interferential stimulation signal may not be able to isolate generation of the neural signal in a single axon type, such that the neural signal generated may be transmitted in several axons towards different parts of the body. However, a velocity of the neural signal may differ in dependence of a part of the body from / to which the neural signal is to be transmitted. Thus, the phase relation between the second interferential stimulation signal and the neural signal may allow blocking or strengthening the neural signal at a particular time at the second location in relation to the nerve. This implies that the second interferential stimulation signal may select which part of the neural signal to strengthen or block such that the stimulation may ensure that neural signals are strengthened / blocked in relation to a particular part of the body.

According to an embodiment, the set of electrodes and the measurement unit are arranged in one or more cuffs configured to be arranged around a nerve for providing stimulation of the nerve.

This implies that accurate control of positioning of the set of electrodes and the measurement unit in relation to the nerve may be provided.

It should be realized that the set of electrodes and the measurement unit may be arranged in any carrier configured to conform to a shape of the brain and/or nerve.

The processing unit may also be arranged in the one or more cuffs and/or the carrier. This implies that the processing unit may easily and accurately control the stimulation such that a delay of control signals may not affect the stimulation. However, it should be realized that the processing unit may alternatively be arranged in a separate carrier, but the processing unit is preferably connected to the stimulation generating unit and the measurement unit through a wired connection.

The device of the first aspect may be used in a method for providing brain stimulation and/or nerve stimulation of a living being, said method comprising: generating at least a first high frequency stimulation signal and a second high frequency stimulation signal for stimulating a brain and/or a nerve of the living being, wherein a difference between a first frequency of the first high frequency stimulation signal and a second frequency of the second high frequency stimulation signal defines a beat frequency; transmitting the first high frequency stimulation signal and the second high frequency stimulation signal into the brain and/or the nerve, wherein the first high frequency stimulation signal and the second high frequency stimulation signal are configured to cause interferential stimulation in the brain and/or the nerve by an interferential stimulation signal having the beat frequency, wherein the interferential stimulation signal is formed through interference by the first high frequency stimulation signal and the second high frequency stimulation signal; acquiring a measurement of at least a neural signal propagating in the brain and/or the nerve; determining a phase relation between the neural signal and the interferential stimulation signal; and controlling brain stimulation and/or nerve stimulation based on the determined phase relation.

Thanks to the method, accurate control of brain and/or nerve stimulation is provided.

The device of the first aspect may be used in a method for controlling stimulation signals in the device according to the first aspect, said method comprising: generating at least a first high frequency stimulation signal and a second high frequency stimulation signal, wherein a difference between a first frequency of the first high frequency stimulation signal and a second frequency of the second high frequency stimulation signal defines a beat frequency; providing the first high frequency stimulation signal and the second high frequency stimulation signal, wherein the first high frequency stimulation signal and the second high frequency stimulation signal are configured to cause interferential stimulation by an interferential stimulation signal having the beat frequency, wherein the interferential stimulation signal is formed through interference by the first high frequency stimulation signal and the second high frequency stimulation signal; acquiring a measurement of at least a neural signal; determining a phase relation between the neural signal and the interferential stimulation signal; and controlling stimulation based on the determined phase relation.

The method relates to controlling generation of signals that may be used for stimulation. The method provides control signals for controlling timing of the high frequency stimulation signals. Thus, the method provides control of a device for generating stimulation signals.

Thanks to the providing control signals for controlling generation of the stimulation signals, the stimulation signals may be controlled so as to fit the measurement of the neural signal.

### Brief description of the drawings

The above, as well as additional objects, features, and advantages of the present inventive concept, will be better understood through the following illustrative and non-limiting detailed description, with reference to the appended drawings. In the drawings like reference numerals will be used for like elements unless stated otherwise.
Fig. 1 is a schematic view of a device according to a first embodiment for brain stimulation.
Fig. 2 shows charts illustrating an interferential stimulation signal formed by a first high frequency stimulation signal and a second high frequency stimulation signal.
Fig. 3 shows charts illustrating separation of neural signal and interferential stimulation signal in frequency domain.
Fig. 4 shows charts illustrating extraction of phase relation between neural signal and interferential stimulation signal according to one approach.
Fig. 5 shows charts illustrating extraction of phase relation between neural signal and interferential stimulation signal according to another approach.
Fig. 6 shows charts illustrating adapting of the interferential stimulation signal based on adapting phase of the first high frequency stimulation signal.
Fig. 7 is a schematic view of a device according to a second embodiment for nerve stimulation.
Fig. 8 shows charts illustrating extracting neural signal and interferential signal according to one approach.
Fig. 9 shows charts illustrating extracting neural signal according to another approach.
Fig. 10 shows charts illustrating interferential stimulation of a nerve.
Fig. 11 shows a chart illustrating an interferential signal comprising a sequence of bursts.
Fig. 12 is a schematic view of a device according to a third embodiment for nerve stimulation illustrating evoked neural signals by the device.
Fig. 13 is a schematic view of a device according to a fourth embodiment for nerve stimulation illustrating calibration and stimulation of evoked neural signals by the device.
Fig. 14 is a flow chart of a method according to an embodiment.

### Detailed description

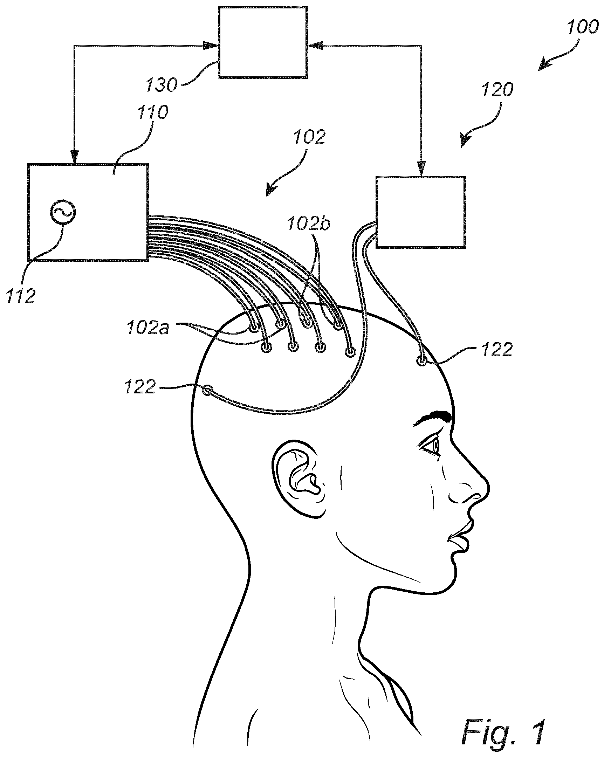

Referring now to Fig. 1, a device 100 for providing brain stimulation of a living being, such as a human being, will be described.

As shown in Fig. 1, the device 100 may be set up to provide a transcranial stimulation. Thus, the device 100 may comprise a set of electrodes 102, which may be configured to be attached to a scalp of a subject such that a stimulation signal may be provided to the brain of the subject through the cranium. However, it should be realized that the device 100 may alternatively be set up to provide stimulation signals in another manner to the brain, such as using electrodes that may be attached to an exposed brain corresponding to a set-up for recording an electrocorticography (ECoG) signal or using electrodes in a neural probe that is to be inserted into brain tissue.

The device 100 may further comprise a stimulation generating unit 110. The stimulation generating unit 110 may be any type of unit which is able to controllably output an electrical signal. The stimulation generating unit 110 may be configured to tune parameters of the output electrical signal so as to control the output electrical signal. For instance, the stimulation generating unit 110 may be configured to tune frequency, phase, and waveform of the electrical signal. The stimulation generating unit 110 may further be configured to generate the electrical signal using selected parameters. The stimulation generating unit 110 may be connected to the set of electrodes 102 and output the generated electrical signal to set of electrodes 102.

The stimulation generating unit 110 may be configured to generate at least a first high frequency stimulation signal and a second high frequency stimulation signal. The stimulation generating unit 110 may further be configured to generate the first high frequency stimulation signal and the second high frequency stimulation signal with a difference between a first frequency of the first high frequency stimulation signal and a second frequency of the second high frequency stimulation signal. The difference between the first frequency and the second frequency defines a beat frequency in interference between the first high frequency stimulation signal and the second high frequency stimulation signal when the first and the second high frequency stimulation signals are output by the set of electrodes 102.

The first and the second frequency are called high frequency in terms of the first and the second frequency being higher than the beat frequency. Also, the first and the second frequency may be sufficiently high so as not to affect or insignificantly affect brain tissue.

For brain stimulation, the first and the second frequency may be larger than 60 Hz, such as in a range of 60 - 100 Hz, or larger than 100 Hz, such as in a range of 150 - 250 Hz or even extending into a range of kHz or MHz frequencies. Such frequencies may differentiate the high frequency stimulation signals from normal frequency content of the neural activity in the brain. However, it should be realized that in some situations, a subject may exhibit higher frequency oscillations in the brain activity, such as in case of epileptic seizures. Thus, the first and the second frequency may be at least 1 kHz or even larger.

The first and the second frequency may preferably not be too high so that a relatively small difference in the frequencies may still be accurately defined.

The beat frequency used may depend on the effect on the brain that is desired. For brain stimulation, the beat frequency may correspond to frequencies of brain waves such that the beat frequency may be in a range of 0.5 - 45 Hz, such as 8 - 12 Hz. Different types of brain waves having different frequencies may thus be stimulated. Although there may not be an exact, well-established definition of different brain waves, brain waves may be referred to as delta waves (0.5 - 3 Hz), theta waves (3 - 8 Hz), alpha waves (8 - 12 Hz), beta waves (12 - 38 Hz) and gamma waves (38 - 42 Hz).

The stimulation generating unit 110 may thus be configured to generate at least a first and a second high frequency stimulation signal. This may be achieved by a single stimulation generator, but it should be realized that the stimulation generating unit 110 may comprise separate stimulation generators for each of the stimulation signals to be generated. Having separate stimulation generators may ensure that generation and output of stimulation signals from the stimulation generators may be individually provided without crosstalk between the stimulation generators.

Each stimulation generator of the stimulation generating unit 110 may comprise a current generator 112. The current generator 112 is configured to generate a high frequency stimulation signal and to output the high frequency stimulation signal to the set of electrodes 102.

The set of electrodes 102 may comprise a plurality of electrodes 102 arranged in relation to the brain. The set of electrodes 102 is configured to receive the first and the second high frequency stimulation signal from the stimulation generating unit 110. Each of the first and the second high frequency stimulation signal is received by a respective pair of electrodes in the set of electrodes 102. As indicated in Fig. 1, the first high frequency stimulation signal may be received by a first pair of electrodes 102a and the second high frequency stimulation signal may be received by a second pair of electrodes 102b.

The first and the second pair of electrodes 102a, 102b may be connected to always receive the first and the second high frequency stimulation signal, respectively. However, the first and second pair of electrodes 102a, 102b may be defined among the set of electrodes 102 when stimulation is to be provided, such that, at different times, e.g., different stimulation sessions, different electrodes among the set of electrodes 102 may be receiving the first and the second high frequency stimulation signal. Thus, a location in relation to the brain may be selected based on selecting which electrodes to be used.

The first and the second pair of electrodes 102a, 102b are configured to transmit the first and the second high frequency stimulation signal into the brain. The first and the second pair of electrodes 102a, 102b are arranged in different locations to the brain such that interference between the first and the second high frequency stimulation signal occurs in the brain to form an interferential stimulation signal having the beat frequency.

The first and the second pair of electrodes 102a, 102b may thus be separately mounted for arranging the first and the second pair of electrodes 102a, 102b in different locations in relation to the brain. However, it should be realized that the first and the second pair of electrodes 102a, 102b may be arranged close to each other to define slightly different locations in relation to the brain. In such case, the first and the second pair of electrodes 102a, 102b may be mounted in a common carrier that may be arranged in relation to the brain. For instance, the set of electrodes 102 may comprise a grid of a plurality of electrodes, from which the pairs of electrodes 102a, 102b to be used may be selected.

The device 100 may further comprise a measurement unit 120. The measurement unit 120 may comprise at least two sensing electrodes 122 configured to detect an electrical signal. The sensing electrodes 122 may be arranged in relation to the brain in a similar manner as the set of electrodes 102. Thus, the sensing electrodes may be attached to the scalp of the subject such that an electrical signal propagating in the brain, such as a neural signal, may be sensed by the sensing electrodes. However, it should be realized that the sensing electrodes 122 may alternatively be attached to an exposed brain corresponding to a set-up for recording an electrocorticography (ECoG) signal or using sensing electrodes in a neural probe that is to be inserted into brain tissue.

The measurement unit 120 may thus be configured to acquire a measurement of an electrical signal propagating in the brain as sensed by the sensing electrodes 122. The measurement unit 120 may acquire a measurement of the neural signal, such as a brain wave, propagating in the brain, as well as a measurement of the first and the second high frequency stimulation signal forming the interferential stimulation signal.

The measurement unit 120 may be configured to output the measurement to a processing unit 130. The processing unit 130 is thus configured to receive the measurement from the measurement unit 120. The processing unit 130, as will be described in further detail below, is configured to determine a phase relation between the neural signal and the interferential stimulation signal. The processing unit 130 is further configured to control brain stimulation based on the determined phase relation.

For instance, the processing unit 130 may be configured to control the stimulation generating unit 110 such that a phase of the interferential stimulation signal is adapted. The phase of the interferential stimulation signal may for instance be controlled to correspond to the phase of the neural signal, such that the interferential stimulation signal may strengthen the neural signal. Alternatively, the phase of the interferential stimulation signal may be controlled to be out-of-phase, such as with a 180° phase difference to the neural signal, for blocking or weakening the neural signal.

The processing unit 130, the stimulation generating unit 110 and the measurement unit 120 may all be arranged in a common housing. The measurement unit 120 may comprise circuitry for acquiring a measurement signal and possibly (pre-)processing the measurement signal, such as providing analog-to-digital conversion of the measurement signal. The stimulation generating unit 110 may also comprise circuitry for generating an electrical signal based on input parameters.

The processing unit 130 may be implemented in a general-purpose processing unit, such as a central processing unit (CPU), which may execute instructions of one or more computer programs in order to implement functionality of the processing unit 130. However, the processing unit 130 may alternatively be implemented as firmware arranged e.g., in an embedded system, or as a specifically designed processing unit, such as an Application-Specific Integrated Circuit (ASIC) or a Field-Programmable Gate Array (FPGA).

Thus, the processing unit 130, the stimulation generating unit 110 and the measurement unit 120 may be implemented as separate units within the common housing and may be configured to communicate by transmitting signals between the units within the housing. However, it should be realized that functions of the processing unit 130, the stimulation generating unit 110 and the measurement unit 120 may even be intertwined, such that parts of the stimulation generating unit 110 and the measurement unit 120 may also be implemented in a common processor also forming the processing unit 130 so that the different units may be defined by different threads within the common processor.

Further, it should be realized that the processing unit 130, the stimulation generating unit 110 and the measurement unit 120 may be arranged in separate physical housings. Whereas the stimulation generating unit 110 and the measurement unit 120 may need to be arranged in close relation to the subject, the processing unit 130 may be remotely arranged. The processing unit 130, the stimulation generating unit 110 and the measurement unit 120 may then be configured to communicate through wired or wireless communication over a computer and/or telecommunication network.

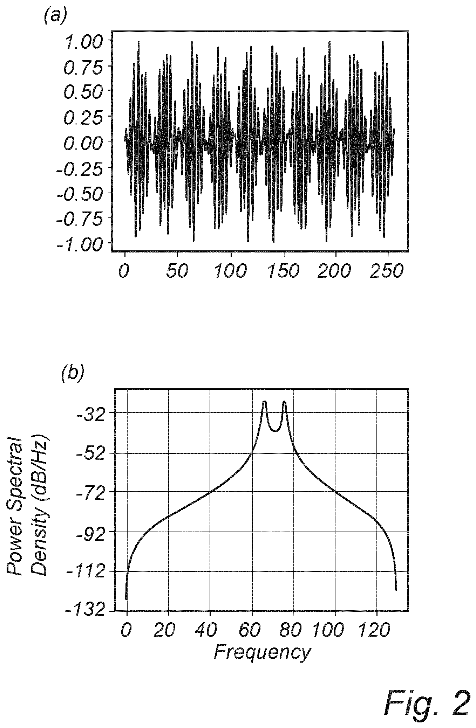

Referring now to Figs 2-6, functions of the stimulation generating unit 110, the measurement unit 120 and the processing unit 130 for controlling brain stimulation will be described in more detail.

In order to provide the interferential stimulation signal at a target frequency, e.g., 10 Hz, the first high frequency stimulation signal may provide a carrier frequency of *f₁* = 65 Hz, and the second high frequency stimulation signal may provide a carrier frequency of *f₂* = 75 Hz, resulting in a beat frequency of *f₂* - *f₁* = 10 Hz of the interferential stimulation signal in the brain tissue.

Fig. 2 shows in chart (a) an interferential stimulation signal formed by interference by the first high frequency stimulation signal and the second high frequency stimulation signal and in chart (b) a power spectrum of the interferential stimulation signal. As shown in Fig. 2, the signal resulting from interferential stimulation by the first high frequency stimulation signal and the second high frequency stimulation signal only comprises peaks corresponding to the first frequency and the second frequency, i.e., frequency components of the two carrier frequencies.

By the first and the second frequencies of the first and the second high frequency stimulation signal being arranged to not affect or insignificantly affect the brain, the first and the second frequencies are outside an electroencephalogram (EEG) power spectrum. This also implies that stimulation artifacts caused by the first and the second frequencies are separated from the target frequency.

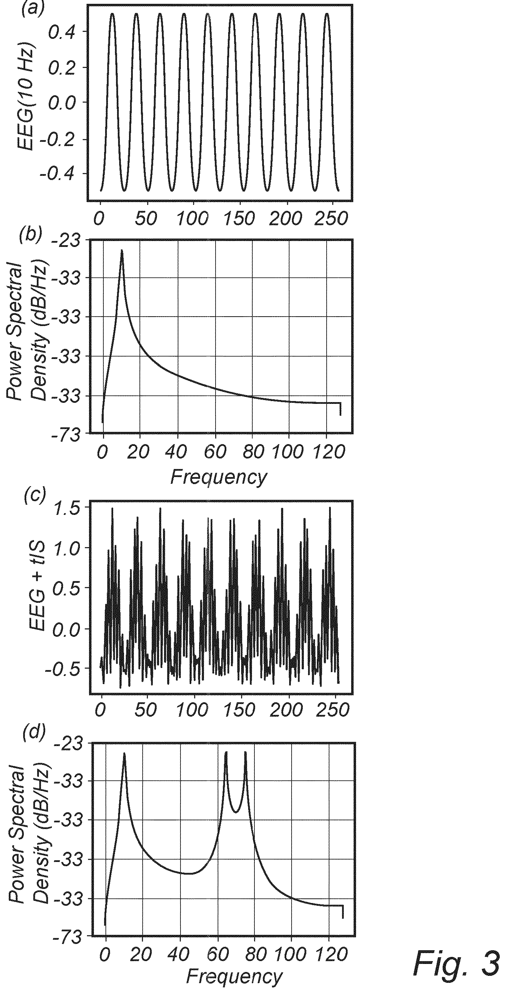

Fig. 3 shows in charts (a) an EEG signal and (b) the power spectrum of the EEG signal when no stimulation is provided. Fig. 3 further shows in chart (c) a recorded signal from interaction of EEG and the interferential stimulation signal and (d) the power spectrum of the recorded signal.

Separation of the neural signal (EEG) and the interferential stimulation signal in frequency domain can be leveraged to extract the phase of the EEG signal and the stimulation signal.

Thus, the neural signal exhibits a neural frequency (target frequency) and the processing unit 130 may be configured to determine the phase relation between the neural signal and the interferential stimulation signal at the neural frequency. The stimulation generating unit 110 may be configured to generate the first high frequency stimulation signal and the second high frequency stimulation signal for defining the beat frequency to equal the neural frequency (target frequency). The measurement unit 120 may further be configured to acquire a measurement of the interferential stimulation signal and the neural signal. The processing unit 130 may further be configured to determine the phase relation between the neural signal and the interferential stimulation signal based on analysis of the neural frequency and at least one of the first frequency and the second frequency of the measurement received from the measurement unit 120.

The processing unit 130 may be configured to perform complex wavelet analysis at the neural frequency of 10 Hz and at one of the first and the second frequency, e.g., at the second frequency of 75 Hz. The processing unit 130 may thus be configured to determine a phase relation, such as a phase error (difference) between the neural signal and the interferential stimulation signal.

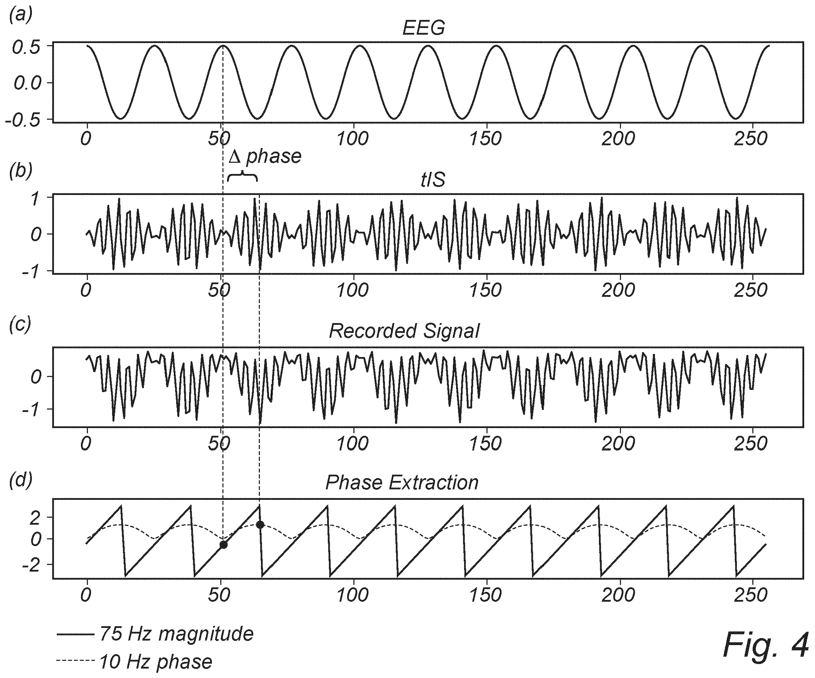

Fig. 4 shows in chart (a) the neural frequency of an EEG, in chart (b) the interferential stimulation signal, in chart (c) signal recorded from sensing electrodes, and in chart (d) wavelet analysis at the neural frequency of 10 Hz and the second frequency of 75 Hz. It should be realized that the signals shown in Fig. 4 are clean signals and may not exactly correspond to actual signals recorded by the measurement unit 120. However, the clean signals are shown here for facilitating illustrating the concept of the present description.

As shown in Fig. 4, chart (d), fiducial points (maxima and minima) from a magnitude signal of the second frequency (75 Hz) and a phase signal of the neural frequency (10 Hz) may be extracted from complex wavelet analysis. The magnitude signal of the second frequency is modulated by the beat frequency allowing the phase of the interferential stimulation signal to be determined. As indicated in Fig. 4 by dashed lines between charts (a) and (d) and charts (b) and (d), a relation between peaks of the neural signal and the interferential stimulation signal may thus be determined providing a phase relation between the neural signal and the interferential stimulation signal.

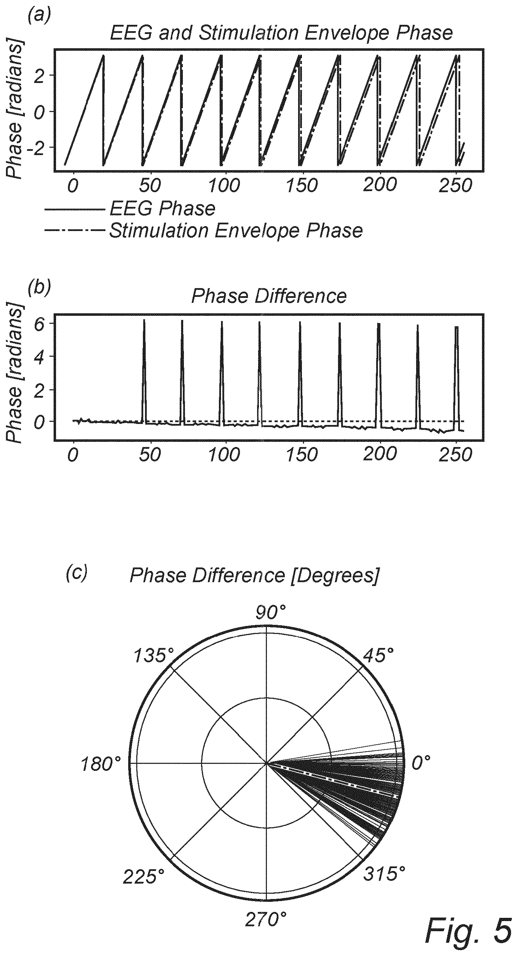

Fig. 5 shows in chart (a) phase signals of both the neural frequency and an envelope of the second frequency (a phase signal corresponding to the magnitude signal in Fig. 4, chart (d)), in chart (b) a phase difference between the phase signals in chart (a), and in chart (c) a polar plot of phase error angles, indicating a resulting phase error angle.

Thus, instead of determining the phase relation based on comparison in Fig. 4, chart (d), the determination of the phase relation may involve extracting the phase of the envelope signal of the second frequency, which allows for a direct phase error computation by subtraction of the phase signals of the neural frequency and the envelope signal of the second frequency.

The determined phase relation may be used for adjusting the phase of the interferential stimulation signal to match a desired phase relation. For instance, it may be desired that the phase of the interferential stimulation signal is in phase (0° difference) with the neural signal for strengthening the neural signal or it may be desired that the phase of the interferential stimulation signal is completely out of phase (180° difference) with the neural signal for weakening / blocking the neural signal.

Given that amplitude modulation of the interferential stimulation signal at the target frequency (equal to the neural frequency of interest) is controlled by the phase differences and starting phases of the first and second frequencies, by precisely adapting the first and the second high frequency stimulation signals, extremely precise phase adaptation of the target amplitude modulated interferential stimulation signal can be achieved. The control of the phase of the interferential stimulation signal may be achieved by controlling (changing) phase of only one of the first high frequency stimulation signal and the second high frequency stimulation signal. Alternatively, the phase of both the first high frequency stimulation signal and the second high frequency stimulation signal may be controlled.

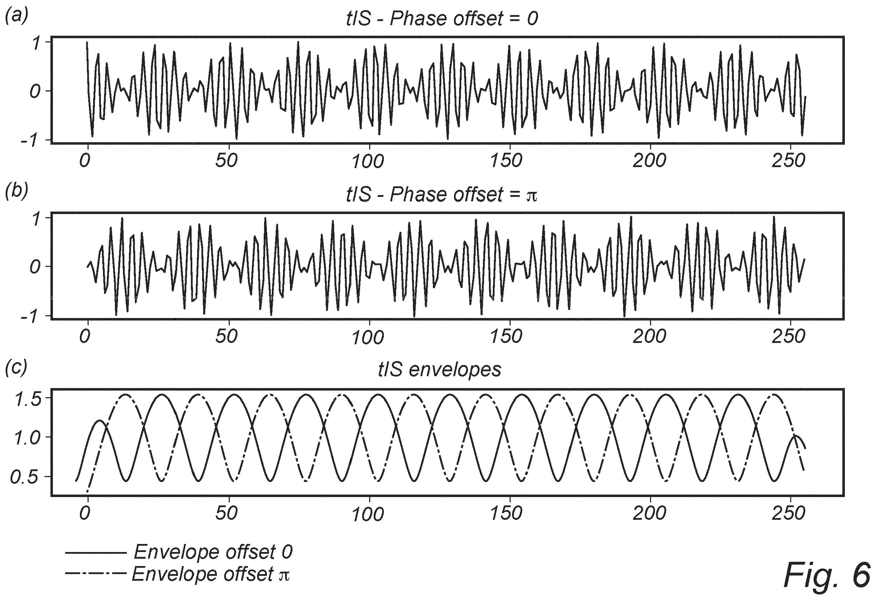

Fig. 6 shows in chart (a) an interferential stimulation signal with both the first and the second high frequency stimulation signal having a starting phase equal to zero, in chart (b) an interferential stimulation signal with the first high frequency stimulation signal having an initial phase offset equal to π and in chart (c) an envelope signal extracted using complex wavelet analysis showing a phase shift of the interferential stimulation signal at the target frequency of 10 Hz equal to the phase offset of the first high frequency stimulation signal. Thus, by changing the initial phase offset of one of the first high frequency stimulation signal and the second high frequency stimulation signal, a phase change of the interferential stimulation signal may be accurately controlled.

The device 100 may be configured to provide continuous stimulation while acquiring measurements, determining phase relations, and adapting the phase of the interferential stimulation signal in dependence of a determined phase relation to the neural signal. Thus, the device 100 may be used for continuously updating the phase of the interferential stimulation signal to adapt to dynamic changes to the neural signal.

In other words, the stimulation generating unit 110 may be configured to generate the first high frequency stimulation signal and the second high frequency stimulation signal during a period of time and the measurement unit 120 may be configured to acquire the measurement during the period of time and the processing unit 130 may be configured to determine the phase relation during the period of time. Thus, during the period of time continuous stimulation may be provided while the phase of the interferential stimulation signal is updated to adapt to dynamic changes to the neural signal.

Moreover, since both brain activity and stimulation are recorded from the same sensor electrodes 122, delays due to communication between devices (processing unit 130, signal generating unit 110, measurement unit 120) are minimized and further compensated due to the continuous adjustments of the phase of the interferential stimulation signal.

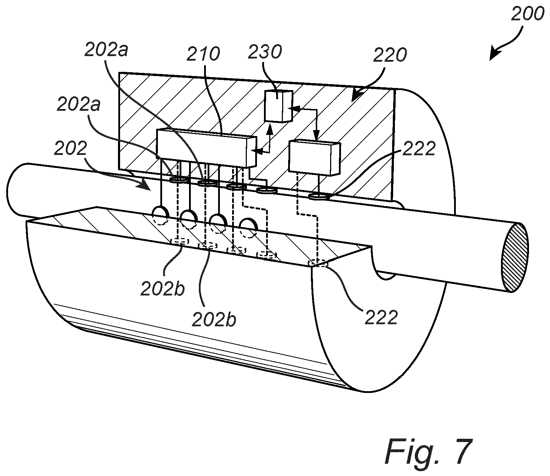

Referring now to Fig. 7, a device 200 for providing nerve stimulation of a living being, such as a human being, will be described. Fig. 7 shows a partially cut-away perspective view of the device 200 to illustrate arrangement of the electrodes in relation to the nerve.

As shown in Fig. 7, the device 200 may be set up to provide nerve stimulation by the device 200 providing a cuff 204 being arranged around the nerve. The device 200 may comprise a set of electrodes 202, which may be arranged in one or more cuffs 204 or may be otherwise configured to be arranged around the nerve.

The device 200 may be configured to provide nerve stimulation such that function of the device 200 may be adapted to suit nerve stimulation. However, like the device 100 for brain stimulation, the device 200 may comprise a stimulation generating unit 210 for generating at least a first high frequency stimulation signal and a second high frequency stimulation signal, a set of electrodes 202 for receiving and transmitting the first and the second high frequency stimulation signal into the nerve for causing interferential stimulation therein by the interferential stimulation signal. The device 200 may further comprise a measurement unit 220 having at least two sensing electrodes 222, which may also be arranged around the nerve, e.g., mounted in the cuff 204. Also, the device 200 may comprise a processing unit 230 configured to determine the phase relation between a neural signal propagating in the nerve and the interferential stimulation signal.

The processing unit 230, the stimulation generating unit 210 and the measurement unit 220 may all be arranged in a common housing, such as the cuff 204. It should be realized that the processing unit 230, the stimulation generating unit 210 and the measurement unit 220 may alternatively be arranged in separate physical housings. Whereas the stimulation generating unit 210 and the measurement unit 220 may need to be arranged in close relation to the subject, the processing unit 230 may be remotely arranged. The processing unit 230, the stimulation generating unit 210 and the measurement unit 220 may then be configured to communicate through wired or wireless communication over a computer and/or telecommunication network.

The first and the second frequency are called high frequency in terms of the first and the second frequency being higher than the beat frequency. Also, the first and the second frequency may be sufficiently high so as not to affect or insignificantly affect nerve tissue or other tissue around the nerve.

For nerve stimulation, the first and the second frequency may be in a range of 500 Hz - 1 MHz, such as 10 - 30 kHz. Such frequencies may differentiate the high frequency stimulation signals from normal frequency content of the neural signals.

The first and the second frequency may preferably not be too high so that a relatively small difference in the frequencies may still be accurately defined.

The beat frequency used may depend on the effect on the nerve that is desired. The beat frequency may correspond to frequencies of neural signals or to repetition frequencies of a sequence of action potentials propagating in the neve, such that the beat frequency may be in a range of 5 Hz - 2 kHz.

The set of electrodes 202 may comprise a first pair of electrodes that may be associated with a first part of the circumference of the nerve 10 and a second pair of electrodes that may be associated with a second part of the circumference of the nerve. The first pair of electrodes may be configured to receive the first high frequency stimulation signal and the second pair of electrodes may be configured to receive the second high frequency stimulation signal. This implies that the portions of the cross-section of the nerve in which the first high frequency stimulation signal and the second high frequency stimulation signal, respectively, define electric fields are quite different so as to allow interferential stimulation deep within the nerve.

However, according to an alternative, the electrodes of the first pair of electrodes may be configured to be alternatingly arranged with the electrodes of the second pair of electrodes around the circumference of the nerve. Different arrangements of the first pair of electrodes and the second pair of electrodes in relation to the nerve may be used depending on a location within the nerve at which stimulation is desired.

According to another alternative shown in Fig. 7, the electrodes of the first pair of electrodes 202a may be displaced in relation to each other along a longitudinal direction of the nerve. The electrodes of the second pair of electrodes 202b may also be displaced in relation to each other along a longitudinal direction of the nerve. The first pair of electrodes 202a may be displaced from the second pair of electrodes 202b along the circumference of the nerve, but the electrodes of the first and second pair of electrodes 202a, 202b may be arranged at common positions in relation to the longitudinal direction of the nerve. This implies that an interferential signal may be provided along the longitudinal direction of the nerve, which may be useful for stimulating the nerve.

As illustrated in Fig. 7, the first pair of electrodes 202a and the second pair of electrodes 202b may be mounted in the cuff 204. The cuff 204 may facilitate arrangement of the first and second pair of electrodes 202a, 202b in contact with a surface of the nerve.

The first pair of electrodes 202a and the second pair of electrodes 202b may be mounted in the cuff 204 with a fixed relation between the electrodes. This implies that the cuff 204 may provide a well-defined relation between the electrodes providing a simple manner of arranging all the electrodes with a desired relationship to each other and to the nerve. However, it should be realized that the electrodes to be used for receiving the first high frequency stimulation signal and the second high frequency stimulation signal may alternatively be dynamically selected among the set of electrodes 202.

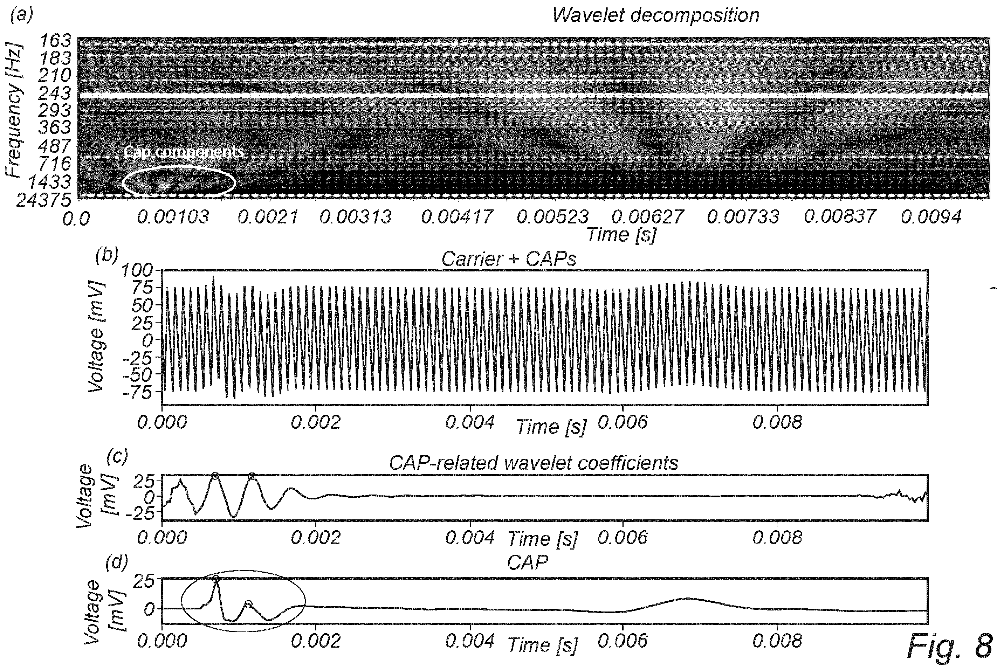

Referring now to Figs 8-10, nerve stimulation used for controlling the interferential stimulation signal in relation to a repetition frequency of a sequence of action potentials propagating in the nerve is discussed.

The nerve may provide neural signals with a periodicity defining a repetition frequency. The first and second high frequency stimulation signals may be configured to define an interferential stimulation signal having a beat frequency corresponding to the neural frequency. In such case, the interferential stimulation signal may be controlled in a similar manner as described above for brain stimulation, even though the frequencies involved may be different.

The electrical activity at neuronal level may be seen as action potentials in an electroneurography (ENG), which may be captured by sensing electrodes 222. The repetition frequency of action potentials is at rates of tens to hundreds of Hz and spectral content of the action potentials may be in range of kHz. The first and the second frequency of the first and the second high frequency stimulation signal, respectively, may be selected so as to differ from the spectral content of action potentials. Thus, the first and the second frequency may for instance be 20 kHz or higher. This implies that the neural signal and the first and second high frequency stimulation signals may be easily separated from each other in an acquired measurement signal, e.g., an ENG, using similar processing as described above in relation to brain stimulation.

The ENG may be recorded using sensing electrodes 222 positioned around the nerve in the cuff 204 and the neural signal may be referred to as a compound action potential (CAP) as the CAP is a summation of activation of a large number of activated nerve fibers.

Phase of the interferential stimulation signal may be extracted using complex wavelet analysis similar to the processing described above for brain stimulation. The neural signal, which may be a response to the interferential stimulation signal, of different fiber types can be extracted by using similar wavelet analysis or adapting shapelets derived based on shape of neuronal responses of different fibers.

Fig. 8 illustrates stimulation based on a sequence of bursts of the first high frequency stimulation signal and the second high frequency stimulation signal defining a repetition frequency of the bursts in the sequence. Fig. 8 shows in chart (a) wavelet decomposition of ENG response comprising a stimulation artifact signal and CAPs (indicated by circle), in chart (b) original recorded ENG signal (artifact + CAPs), in chart (c) wavelet coefficients related to CAPs, and in chart (d) synthetic CAPs. The circle in chart (d) indicate neural response from stimulation and dots in charts (c) and (d) indicate wavelet coefficients and corresponding peaks in CAP. The charts in Fig. 8 are based on simulation using a first frequency of 20 kHz and a second frequency of 20.01 kHz, introducing a synthetic neural response.

As shown in Fig. 8, the stimulation phase of 20 kHz is identified and evoked neural response following this stimulation is extracted using wavelets.

Based on extracting the phase of the interferential stimulation signal and the neural signal, the interferential stimulation signal may be shifted such that the interferential stimulation signal may have a different offset with respect to phase of the neural response, i.e., phase of the activation of different fiber types in the CAP response.

Thus, neural activity can be identified from an original signal comprising stimulation artifact by wavelet decomposition at different frequencies. Once coefficients related to CAPs are identified, a peak extraction algorithm can be used to identify the neural response and its phase with respect to the interferential stimulation signal. In this case, complex Morlet wavelets were used for decomposition, which results in additional peaks at beginning of a signal, but other wavelets may be used for improving detection.

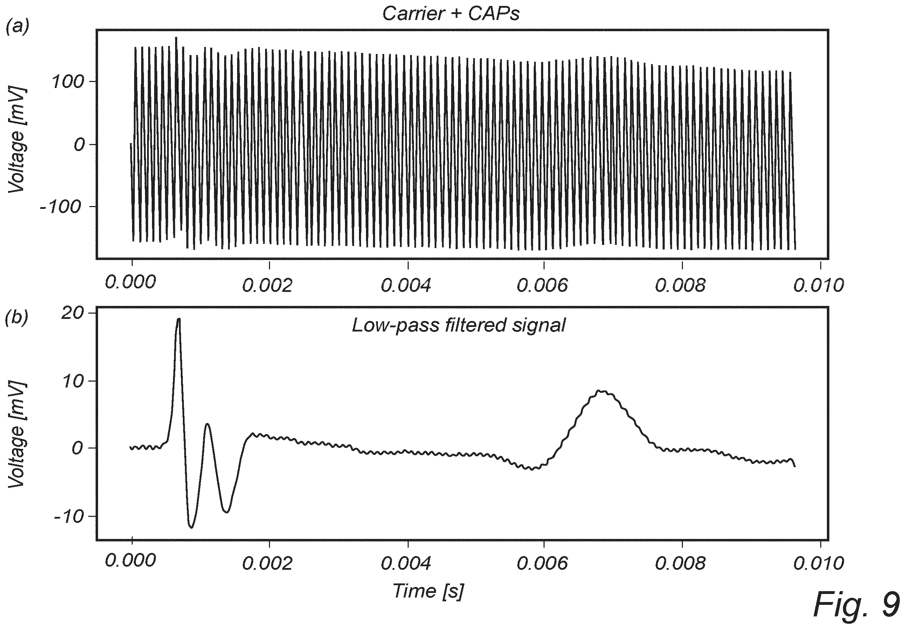

Referring now to Fig. 9, another method for extracting CAPs from the original signal is described. Such method comprises using a low pass filter to remove the stimulation artifacts. The processing may thus exploit a fact that the first and the second frequency are at higher frequencies compared to the CAPs.

Fig. 9 shows in chart (a) an original ENG signal comprising stimulation artifacts and CAPs and in chart (b) a low pass filtered signal comprising only CAPs, showing that the CAPs are isolated. In the case shown in Fig. 9, the original signal comprises a stimulation artifact based on the first high frequency stimulation signal having a first frequency of 20 kHz and the second high frequency stimulation signal having a second frequency of 20.01 kHz and the original signal is low pass filtered at 5 kHz to reveal the CAPs.

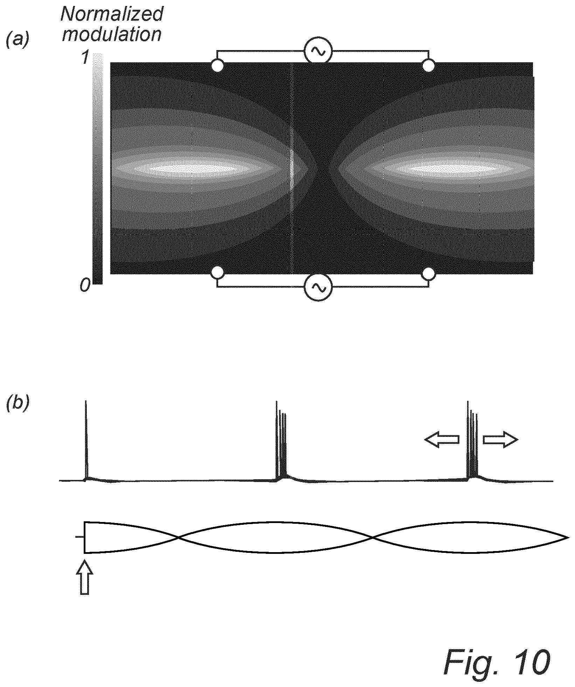

Fig. 10 illustrates the effect of interferential stimulation on a longitudinal cross-section of a nerve. Fig. 10 shows in part (a) how effects of interferential stimulation based on a first pair of electrodes arranged at one side of the nerve (top side in Fig. 10) and a second pair of electrodes arranged at an opposite side of the nerve (bottom side in Fig. 10) are distributed along the fiber. Fig. 10 shows in part (b) activation patterns located in central part of the nerve. A pattern of firing action potentials is shown above an amplitude modulated interferential stimulation signal, showing synchronization between the interferential stimulation signal and the neural signals.

Neural response corresponds to a maximum of the amplitude modulation of the interferential stimulation signal. Hence, by detecting the phase difference between the phase of the interferential stimulation signal and the neural response (captured as CAP), the phase of the interferential stimulation signal can be shifted back and forth in time, as indicated with arrows in Fig. 10 (b).

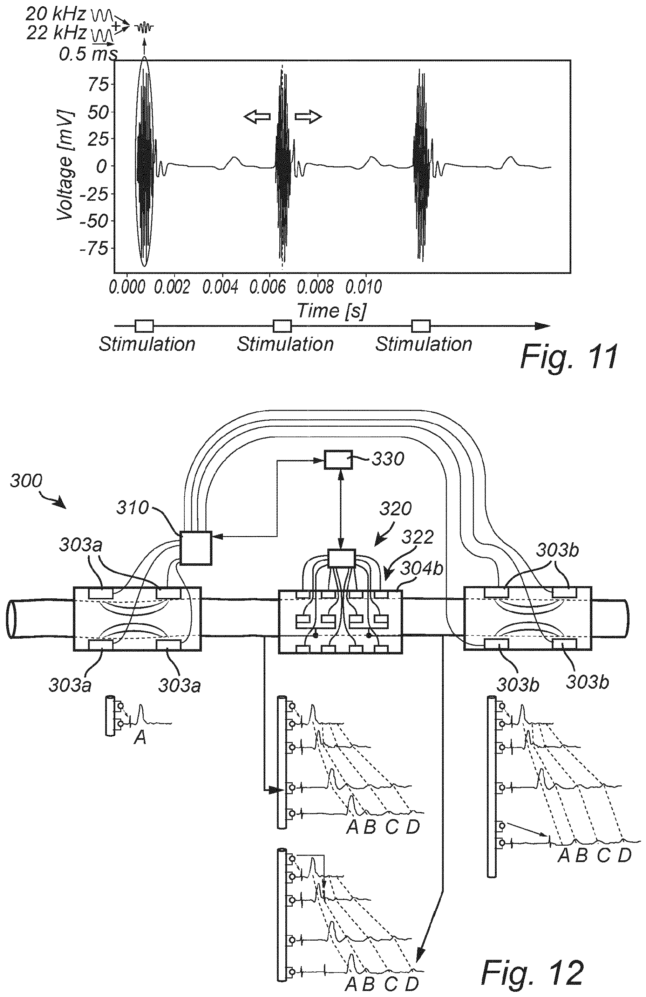

Referring now to Fig. 11, another embodiment for nerve stimulation will be described.

In this embodiment, nerve stimulation is again directed to stimulation of neural signals with a periodicity defining a repetition frequency. Thus, the neural signal exhibits a neural frequency. However, in this case, the nerve stimulation is provided as intermittent interferential stimulation such that the device 200 is not configured to output a continuous stimulation signal but rather outputs a sequence of bursts.

Thus, the stimulation generating unit 210 is configured to generate a sequence of bursts of the first high frequency stimulation signal and the second high frequency stimulation signal defining a repetition frequency of the bursts in the sequence.

Fig. 11 shows interferential stimulation via a sequence of short bursts, followed by evoked CAPs. The short interferential pulses are in this case generated by the first high frequency stimulation signal having a first frequency of 20 kHz and the second high frequency stimulation signal having a second frequency of 22 kHz, which evokes a neural response around 1 ms after start of stimulation.

The processing unit 230 may be configured to determine a phase difference between onset of a burst of the interferential stimulation and the CAPs. Based on this determination, phase of the interferential stimulation signal may be adapted such that the onset of the interferential stimulation may be changed, as illustrated by arrows in Fig. 11.

Referring now to Fig. 12, yet another embodiment for nerve stimulation will be described.

Fig. 12 discloses a device 300 comprising a plurality of cuffs 304a, 304b, 304c, although it should be realized that a single, large cuff could be used instead or that the electrodes may be arranged in relation to the nerve in another manner. The device 300 comprises a set of electrodes 302 comprising a first sub-set of electrodes 303a and a second sub-set of electrodes 303b. The first sub-set of electrodes 303a is configured to be arranged at a first location in relation to the nerve. As shown in Fig. 12, the first sub-set of electrodes 303a is arranged in a first cuff 304a. The second sub-set of electrodes 303b is configured to be arranged at a second location in relation to the nerve. As shown in Fig. 12, the second sub-set of electrodes 303b is arranged in a third cuff 304c.

Each sub-set of electrodes 303a, 303b may be configured to receive high frequency stimulation signals for causing interferential stimulation in the nerve. The first sub-set of electrodes 303a may be configured to receive the first high frequency stimulation signal and the second high frequency stimulation signal for forming a first interferential stimulation signal in the nerve at the first location. The second sub-set of electrodes 303b may be configured to receive a third high frequency stimulation signal and a fourth high frequency stimulation signal for forming a second interferential stimulation signal in the nerve at the second location.

Thus, the device 300 may comprise a stimulation generating unit 310 which sis configured to generate a first, second, third and fourth high frequency stimulation signal forming two different pairs of high frequency stimulation signals. The pairs of high frequency stimulation signals may have the same frequencies, such that the same beat frequency may be defined for both the first and the second interferential stimulation signal.

The electrodes in each sub-set of electrodes 303a, 303b may be arranged in any of the different manners discussed above in relation to Fig. 7. As shown in Fig. 12, the sub-set of electrodes 303a, 303b may each define at least two pairs of electrodes for receiving the two high frequency stimulation signals, wherein the electrodes in the pairs of electrodes are displaced in relation to each other along a longitudinal direction of the nerve.

Thanks to having two sub-sets of electrodes 303a, 303b arranged at different locations along a longitudinal direction of the nerve, the first and the second interferential stimulation may be used for providing a combined effect on the nerve. This enables more versatile use of stimulation of the nerve.

Thus, the first interferential stimulation signal may activate a neural signal and the second interferential stimulation signal may strengthen or weaken part of the neural signal triggered by the first interferential stimulation signal. In this way, only a selected type of peripheral nerve fibers may be activated and only in a selected region targeted with interferential stimulation. Thanks to anatomical organization of different fiber types and differences in propagation speed of different fibers, spatio-temporal selectivity of nerve fiber activation can be achieved.

The first interferential stimulation signal may thus evoke CAPs in the nerve. The evoked CAPs (neural signals) propagating in the nerve may be detected by a measurement unit 320 comprising a grid of sensing electrodes 322 arranged in a second cuff 304b, between the first cuff 304a and the third cuff 304c.

The measurement unit 320 may thus capture an ENG response of the first interferential stimulation signal. The processing unit 330 may be configured to identify a phase of the first interferential stimulation signal and a phase of the CAP response of the targeted fiber types. Based on a known arrangement of the second sub-set of electrodes 303b in relation to the grid of sensing electrodes 322, the phase of the second interferential stimulation signal may be controlled. The second interferential stimulation signal may thus provide a phase angle such that the second interferential stimulation signal may strengthen or weaken the neural signal passing the third cuff 304c at a particular time. Since timing of the neural signal at the third cuff 304c may depend on a nerve fiber type, the device 300 may be able to block undesired neural signals going towards a part of a body while allowing desired neural signal going towards another part of the body to pass. Thus, even though activation of CAPs may not perfectly selectively stimulate a small region of the nerve, a relatively broad activation of neural signals may be further controlled by the second interferential stimulation signal.

As shown in timing charts of Fig. 12, the first interferential stimulation signal may evoke a CAP response at a first point in time, the grid of sensing electrodes 322 may detect propagation of signals at two further points in time and the second interferential stimulation signal may block a part A of the CAP response while allowing parts B, C, and D of the CAP response to pass at yet a later point in time when the neural signal reaches the location of the third cuff 304c.

Providing interferential stimulation at multiple different locations along a peripheral nerve and providing stimulation in a synchronous manner enables enhancement, reduction, or more precise steering of stimulation towards a target.

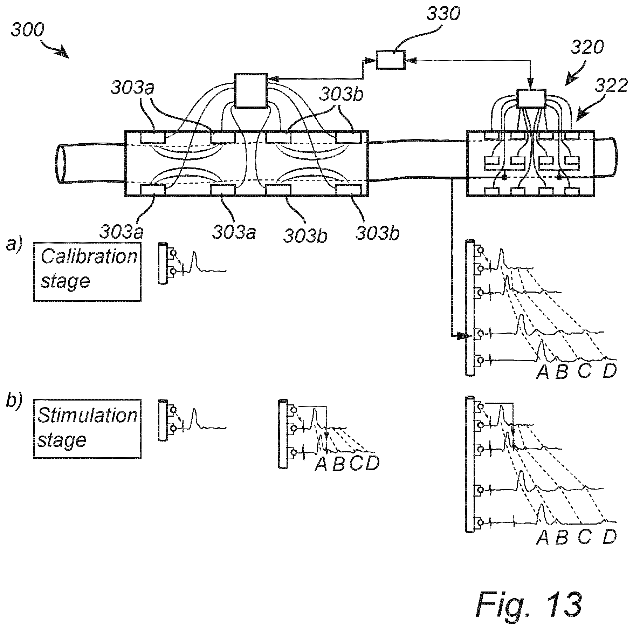

Referring now to Fig. 13, another embodiment for providing synchronized stimulation by a first and a second sub-set of electrodes 303a, 303b will be described. As illustrated in Fig. 13, it is not necessary to have the grid of sensing electrodes 322 arranged between the first sub-set of electrodes 303a and the second sub-set of electrodes 303b.

As shown in Fig. 13, the first and the second sub-set of electrodes 303a, 303b may be arranged at different longitudinal positions in a common cuff. The grid of sensing electrodes 322 is arranged displaced from the first and the second sub-set of electrodes 303a, 303b along a longitudinal direction of the nerve in a direction of propagation of the neural signal to be affected.

In this case, timing of the first and the second interferential stimulation signals may thus be controlled based on calibration. The grid of sensing electrodes 322 may thus be configured to sense a phase of the neural signal evoked by the first interferential stimulation signal during a calibration stage, illustrated in chart (a) of Fig. 13. In the calibration stage, speed of responses of different fiber types to stimulation using the first interference stimulation signal may be determined.

In a stimulation stage, illustrated in chart (b) of Fig. 13, a phase of the second interference stimulation signal is controlled to provide a desired effect on the CAP response induced by the first interference stimulation signal, e.g., the blocking of a part of the signal.

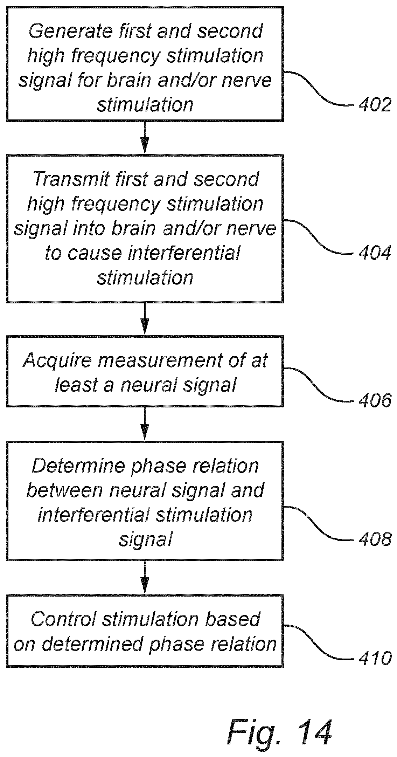

Referring now to Fig. 14, a method for providing brain stimulation and/or nerve stimulation will be described.

The method comprises generating 402 at least a first high frequency stimulation signal and a second high frequency stimulation signal for stimulating a brain and/or a nerve of the living being. The difference between a first frequency of the first high frequency stimulation signal and a second frequency of the second high frequency stimulation signal defines a beat frequency.

The method further comprises transmitting 404 the first high frequency stimulation signal and the second high frequency stimulation signal into the brain and/or the nerve. This causes interferential stimulation in the brain and/or the nerve by an interferential stimulation signal having the beat frequency, wherein the interferential stimulation signal is formed through interference by the first high frequency stimulation signal and the second high frequency stimulation signal.

The method further comprises acquiring 406 a measurement of at least a neural signal propagating in the brain and/or the nerve.

The method further comprises determining 408 a phase relation between the neural signal and the interferential stimulation signal.

Finally, the method further comprises controlling 410 brain stimulation and/or nerve stimulation based on the determined phase relation.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A device (100; 200; 300) for providing brain stimulation and/or nerve stimulation of a living being, said device (100; 200; 300) comprising:
a stimulation generating unit (110; 210; 310) configured to generate at least a first high frequency stimulation signal and a second high frequency stimulation signal for stimulating a brain and/or a nerve of the living being, wherein a difference between a first frequency of the first high frequency stimulation signal and a second frequency of the second high frequency stimulation signal defines a beat frequency;
a set of electrodes (102; 202; 302, 303a, 303b) configured to receive the first high frequency stimulation signal and the second high frequency stimulation signal and configured to be arranged in relation to the brain and/or the nerve for transmitting the first high frequency stimulation signal and the second high frequency stimulation signal into the brain and/or the nerve, wherein the set of electrodes (102; 202; 302, 303a, 303b) are configured to cause interferential stimulation in the brain and/or the nerve by an interferential stimulation signal having the beat frequency, wherein the interferential stimulation signal is formed through interference by the first high frequency stimulation signal and the second high frequency stimulation signal;
a measurement unit (120; 220; 320), comprising at least two sensing electrodes (122; 222; 322), wherein the measurement unit (120; 220; 230) is configured to acquire a measurement of at least a neural signal propagating in the brain and/or the nerve;
a processing unit (130; 230; 330), configured to receive the measurement from the measurement unit (120; 220; 320) and configured to determine a phase relation between the neural signal and the interferential stimulation signal and configured to control brain stimulation and/or nerve stimulation based on the determined phase relation.

2. The device according to claim 1, wherein the neural signal exhibits a neural frequency and wherein the processing unit (130; 230; 330) is configured to determine the phase relation at the neural frequency.

3. The device according to claim 2, wherein the stimulation generating unit (110; 210) is configured to generate the first high frequency stimulation signal and the second high frequency stimulation signal for defining the beat frequency to equal the neural frequency.

4. The device according to claim 2 or 3, wherein the measurement unit (120; 220; 320) is configured to acquire a measurement of the interferential stimulation signal and the neural signal, wherein the processing unit (130; 230; 330) is configured to determine the phase relation based on analysis of the neural frequency and at least one of the first frequency of the first high frequency stimulation signal or the second frequency of the second high frequency stimulation signal.

5. The device according to claim 4, wherein the processing unit (130; 230; 330) is configured to determine phase information of the interferential stimulation signal based on analysis of at least one of the first frequency of the first high frequency stimulation signal or the second frequency of the second high frequency stimulation signal.

6. The device according to claim 2, wherein the stimulation generating unit (210) is configured to generate a sequence of bursts of the first high frequency stimulation signal and the second high frequency stimulation signal defining a repetition frequency of the bursts in the sequence, wherein the processing unit (230) is configured to control a phase relation such that onset of a burst of the interferential stimulation signal is in desired phase relation with the neural signal at the neural frequency.

7. The device according to any one of the preceding claims, wherein the stimulation generating unit (110; 210; 310) is configured to generate the first high frequency stimulation signal and the second high frequency stimulation signal during a period of time and wherein the measurement unit (120; 220; 320) is configured to acquire the measurement during the period of time and the processing unit (130; 230; 330) is configured to determine the phase relation during the period of time.

8. The device according to any one of the preceding claims, wherein the processing unit (130; 230; 330) is configured to control the stimulation generating unit (110; 210; 310) to adapt at least one of the first high frequency stimulation signal or the second high frequency stimulation signal in order to change the phase relation between the neural signal and the interferential stimulation signal.

9. The device according to claim 1, wherein the stimulation generating unit (310) is configured to generate a third high frequency stimulation signal and a fourth high frequency stimulation signal for stimulating a brain and/or a nerve of the living being, wherein a difference between a third frequency of the third high frequency stimulation signal and a fourth frequency of the fourth high frequency stimulation signal defines a beat frequency;
wherein the set of electrodes (302) comprises a first sub-set of electrodes (303a) configured to receive the first high frequency stimulation signal and the second high frequency stimulation signal and configured to be arranged at a first location in relation to the brain and/or the nerve for causing interferential stimulation by a first interferential stimulation signal forming said interferential signal, and wherein the device (300) further comprises a second sub-set of electrodes (303b) configured to receive the third high frequency stimulation signal and the fourth stimulation signal and configured to be arranged at a second location different from the first location in relation to the brain and/or the nerve for transmitting the third high frequency stimulation signal and the fourth high frequency stimulation signal into the brain and/or the nerve, wherein the second set of electrodes (303b) are configured to cause interferential stimulation in the brain and/or the nerve by a second interferential stimulation signal having the beat frequency, wherein the second interferential stimulation signal is formed through interference by the third high frequency stimulation signal and the fourth high frequency stimulation signal;
wherein the processing unit (330) is configured to determine a phase relation between the neural signal and the first interferential stimulation signal and wherein the processing unit (330) is further configured to control the second interferential stimulation signal based on the determined phase relation.

10. The device according to claim 9, wherein the processing unit (330) is configured to control the second interferential stimulation signal for controlling blocking or strengthening a neural signal triggered by the first interferential stimulation signal.

11. The device according to any one of the preceding claims, wherein the set of electrodes (202; 302, 303a, 303b) and the measurement unit (220; 320) are arranged in one or more cuffs (204; 304a, 304b, 304c) configured to be arranged around a nerve for providing stimulation of the nerve.

## Patentansprüche

1. Vorrichtung (100; 200; 300) zum Bereitstellen von Hirnstimulation und/oder Nervenstimulation eines Lebewesens, wobei die Vorrichtung (100; 200; 300) umfasst:
eine Stimulationserzeugungseinheit (110; 210; 310), die dazu konfiguriert ist, mindestens ein erstes Hochfrequenz-Stimulationssignal und ein zweites Hochfrequenz-Stimulationssignal zum Stimulieren eines Gehirns und/oder eines Nervs des Lebewesens zu erzeugen, wobei eine Differenz zwischen einer ersten Frequenz des ersten Hochfrequenz-Stimulationssignals und einer zweiten Frequenz des zweiten Hochfrequenz-Stimulationssignals eine Schlagfrequenz definiert;
eine Menge von Elektroden (102; 202; 302, 303a, 303b), die dazu konfiguriert sind, das erste Hochfrequenz-Stimulationssignal und das zweite Hochfrequenz-Stimulationssignal zu empfangen, und dazu konfiguriert sind, im Verhältnis zu dem Gehirn und/oder Nerv angeordnet zu sein, um das erste Hochfrequenz-Stimulationssignal und das zweite Hochfrequenz-Stimulationssignal in das Gehirn und/oder den Nerv zu übertragen, wobei die Menge von Elektroden (102; 202; 302, 303a, 303b) dazu konfiguriert ist, eine Interferenzstimulation in dem Gehirn und/oder Nerv durch ein Interferenzstimulationssignal, das die Schlagfrequenz aufweist, zu verursachen, wobei das Interferenzstimulationssignal über eine Interferenz durch das erste Hochfrequenz-Stimulationssignal und das zweite Hochfrequenz-Stimulationssignal gebildet wird;
eine Messeinheit (120; 220; 320), die mindestens zwei Abtastelektroden (122; 222; 322) umfasst, wobei die Messeinheit (120; 220; 230) dazu konfiguriert ist, eine Messung mindestens eines neuronalen Signals, das sich in dem Gehirn und/oder Nerv ausbreitet, zu erfassen;
eine Verarbeitungseinheit (130; 230; 330), die dazu konfiguriert ist, die Messung aus der Messeinheit (120; 220; 320) zu empfangen, und dazu konfiguriert ist, eine Phasenbeziehung zwischen dem neuronalen Signal und dem Interferenzstimulationssignal zu bestimmen, und dazu konfiguriert ist, die Hirnstimulation und/oder Nervenstimulation basierend auf der bestimmten Phasenbeziehung zu bestimmen.

2. Vorrichtung nach Anspruch 1, wobei das neuronale Signal eine neuronale Frequenz aufweist, und wobei die Verarbeitungseinheit (130; 230; 330) dazu konfiguriert ist, die Phasenbeziehung auf der neuronalen Frequenz zu bestimmen.

3. Vorrichtung nach Anspruch 2, wobei die Stimulationserzeugungseinheit (110; 210) dazu konfiguriert ist, das erste Hochfrequenz-Stimulationssignal und das zweite Hochfrequenz-Stimulationssignal dazu zu erzeugen, die Schlagfrequenz gleich der neuronalen Frequenz zu definieren.

4. Vorrichtung nach Anspruch 2 oder 3, wobei die Messeinheit (120; 220; 320) dazu konfiguriert ist, eine Messung des Interferenzstimulationssignals und des neuronalen Signals zu erfassen, wobei die Verarbeitungseinheit (130; 230; 330) dazu konfiguriert ist, die Phasenbeziehung basierend auf der Analyse der neuronalen Frequenz und mindestens einer von der ersten Frequenz des ersten Hochfrequenz-Stimulationssignals oder der zweiten Frequenz des zweiten Hochfrequenz-Stimulationssignals zu bestimmen.

5. Vorrichtung nach Anspruch 4, wobei die Verarbeitungseinheit (130; 230; 330) dazu konfiguriert ist, Phaseninformationen des Interferenzstimulationssignals basierend auf der Analyse mindestens einer von der ersten Frequenz des ersten Hochfrequenz-Stimulationssignals oder der zweiten Frequenz des zweiten Hochfrequenz-Stimulationssignals zu bestimmen.

6. Vorrichtung nach Anspruch 2, wobei die Stimulationserzeugungseinheit (210) dazu konfiguriert ist, eine Sequenz von Bursts des ersten Hochfrequenz-Stimulationssignals und des zweiten Hochfrequenz-Stimulationssignals zu erzeugen, die eine Wiederholfrequenz der Bursts in der Sequenz definiert, wobei die Verarbeitungseinheit (230) dazu konfiguriert ist, eine Phasenbeziehung derart zu regeln, dass sich der Beginn eines Bursts des Interferenzstimulationssignals in der gewünschten Phasenbeziehung mit dem neuronalen Signal auf der neuronalen Frequenz befindet.

7. Vorrichtung nach einem der vorhergehenden Ansprüche, wobei die Stimulationserzeugungseinheit (110; 210; 310) dazu konfiguriert ist, das erste Hochfrequenz-Stimulationssignal und das zweite Hochfrequenz-Stimulationssignal während eines Zeitraums zu erzeugen, und wobei die Messeinheit (120; 220; 320) dazu konfiguriert ist, die Messung während des Zeitraums zu erfassen, und die Verarbeitungseinheit (130; 230; 330) dazu konfiguriert ist, die Phasenbeziehung während des Zeitraums zu bestimmen.

8. Vorrichtung nach einem der vorhergehenden Ansprüche, wobei die Verarbeitungseinheit (130; 230; 330) dazu konfiguriert ist, die Stimulationserzeugungseinheit (110; 210; 310) zu regeln, um mindestens eines von dem ersten Hochfrequenz-Stimulationssignal oder dem zweiten Hochfrequenz-Stimulationssignal anzupassen, um die Phasenbeziehung zwischen dem neuronalen Signal und dem Interferenzstimulationssignal zu ändern.

9. Vorrichtung nach Anspruch 1, wobei die
Stimulationserzeugungseinheit (310) dazu konfiguriert ist, ein drittes Hochfrequenz-Stimulationssignal und ein viertes Hochfrequenz-Stimulationssignal zum Stimulieren eines Gehirns und/oder eines Nervs des Lebewesens zu erzeugen, wobei eine Differenz zwischen einer dritten Frequenz des dritten Hochfrequenz-Stimulationssignals und einer vierten Frequenz des vierten Hochfrequenz-Stimulationssignals eine Schlagfrequenz definiert;
wobei die Menge von Elektroden (302) eine erste Teilmenge von Elektroden (303a) umfasst, die dazu konfiguriert sind, das erste Hochfrequenz-Stimulationssignal und das zweite Hochfrequenz-Stimulationssignal zu empfangen, und dazu konfiguriert sind, an einer ersten Stelle im Verhältnis zu dem Gehirn und/oder Nerv angeordnet zu sein, um eine Interferenzstimulation durch ein erstes Interferenzstimulationssignal zu verursachen, welches das Interferenzsignal bildet , und wobei die Vorrichtung (300) ferner eine zweite Teilmenge von Elektroden (303b) umfasst, die dazu konfiguriert sind, das dritte Hochfrequenz-Stimulationssignal und das vierte Stimulationssignal zu empfangen, und dazu konfiguriert sind, an einer zweiten Stelle, die anders als die erste Stelle ist, im Verhältnis zu dem Gehirn und/oder Nerv angeordnet zu sein, um das dritte Hochfrequenz-Stimulationssignal und das vierte Hochfrequenz-Stimulationssignal in das Gehirn und/oder den Nerv zu übertragen, wobei die zweite Menge von Elektroden (303b) dazu konfiguriert ist, eine Interferenzstimulation in dem Gehirn und/oder Nerv durch ein zweites Interferenzstimulationssignal, das die Schlagfrequenz aufweist, zu verursachen, wobei das zweite Interferenzstimulationssignal über eine Interferenz durch das dritte Hochfrequenz-Stimulationssignal und das vierte Hochfrequenz-Stimulationssignal gebildet wird;
wobei die Verarbeitungseinheit (330) dazu konfiguriert ist, eine Phasenbeziehung zwischen dem neuronalen Signal und dem ersten Interferenzstimulationssignal zu bestimmen, und wobei die Verarbeitungseinheit (330) ferner dazu konfiguriert ist, das zweite Interferenzstimulationssignal basierend auf der bestimmten Phasenbeziehung zu regeln.

10. Vorrichtung nach Anspruch 9, wobei die Verarbeitungseinheit (330) dazu konfiguriert ist, das zweite Interferenzstimulationssignal zu regeln, um das Blockieren oder Verstärken eines neuronalen Signals, das durch das erste Interferenzstimulationssignal ausgelöst wird, zu regeln.

11. Vorrichtung nach einem der vorhergehenden Ansprüche, wobei die Menge von Elektroden (202; 302, 303a, 303b) und die Messeinheit (220; 320) in einer oder mehreren Manschetten (204; 304a, 304b, 304c) angeordnet sind, die dazu konfiguriert sind, um einen Nerv herum angeordnet zu sein, um eine Stimulation des Nervs bereitzustellen.

## Revendications

1. Dispositif (100 ; 200 ; 300) pour la stimulation cérébrale et/ou la stimulation nerveuse d'un être vivant, ledit dispositif (100 ; 200 ; 300) comprenant :
une unité de génération de stimulation (110 ; 210 ; 310) configurée pour générer au moins un premier signal de stimulation à haute fréquence et un deuxième signal de stimulation à haute fréquence pour stimuler le cerveau et/ou un nerf de l'être vivant, dans lequel une différence entre une première fréquence du premier signal de stimulation à haute fréquence et une deuxième fréquence du deuxième signal de stimulation à haute fréquence définissent une fréquence de battement ;
un ensemble d'électrodes (102 ; 202 ; 302, 303a, 303b) configurées pour recevoir le premier signal de stimulation à haute fréquence et le deuxième signal de stimulation à haute fréquence et configurées pour être disposées par rapport au cerveau et/ou au nerf afin de transmettre le premier signal de stimulation à haute fréquence et le deuxième signal de stimulation à haute fréquence dans le cerveau et/ou le nerf, dans lequel l'ensemble d'électrodes (102 ; 202 ; 302, 303a, 303b) est configuré pour provoquer une stimulation interférentielle dans le cerveau et/ou le nerf par un signal de stimulation interférentielle ayant la fréquence de battement, dans lequel le signal de stimulation interférentielle est formé par interférence par le premier signal de stimulation à haute fréquence et le deuxième signal de stimulation à haute fréquence ;
une unité de mesure (120 ; 220 ; 320), comprenant au moins deux électrodes de détection (122 ; 222 ; 322), dans lequel l'unité de mesure (120 ; 220; 230) est configurée pour acquérir une mesure d'au moins un signal neuronal se propageant dans le cerveau et/ou le nerf ;
une unité de traitement (130 ; 230 ; 330), configurée pour recevoir la mesure de l'unité de mesure (120 ; 220 ; 320) et configurée pour déterminer une relation de phase entre le signal neuronal et le signal de stimulation interférentielle et configurée pour commander la stimulation cérébrale et/ou nerveuse sur la base de la relation de phase déterminée.

2. Dispositif selon la revendication 1, dans lequel le signal neuronal présente une fréquence neuronale et dans lequel l'unité de traitement (130 ; 230 ; 330) est configurée pour déterminer la relation de phase à la fréquence neuronale.

3. Dispositif selon la revendication 2, dans lequel l'unité de génération de stimulation (110 ; 210) est configurée pour générer le premier signal de stimulation à haute fréquence et le deuxième signal de stimulation à haute fréquence afin de définir la fréquence de battement comme égale à la fréquence neuronale.

4. Dispositif selon la revendication 2 ou 3, dans lequel l'unité de mesure (120 ; 220 ; 320) est configurée pour acquérir une mesure du signal de stimulation interférentielle et du signal neuronal, dans lequel l'unité de traitement (130 ; 230 ; 330) est configurée pour déterminer la relation de phase sur la base de l'analyse de la fréquence neuronale et d'au moins la première fréquence du premier signal de stimulation à haute fréquence ou la deuxième fréquence du deuxième signal de stimulation à haute fréquence.

5. Dispositif selon la revendication 4, dans lequel l'unité de traitement (130 ; 230 ; 330) est configurée pour déterminer les informations de phase du signal de stimulation interférentielle sur la base de l'analyse d'au moins une de la première fréquence du premier signal de stimulation à haute fréquence ou la deuxième fréquence du deuxième signal de stimulation à haute fréquence.

6. Dispositif selon la revendication 2, dans lequel l'unité de génération de stimulation (210) est configurée pour générer une séquence de salves du premier signal de stimulation à haute fréquence et du deuxième signal de stimulation à haute fréquence, définissant une fréquence de répétition des salves dans la séquence, dans lequel l'unité de traitement (230) est configurée pour établir une relation de phase telle que le début d'une salve du signal de stimulation interférentielle soit en phase avec le signal neuronal à la fréquence neuronale.

7. Dispositif selon une quelconque des revendications précédentes, dans lequel l'unité de génération de stimulation (110 ; 210 ; 310) est configurée pour générer le premier signal de stimulation à haute fréquence et le deuxième signal de stimulation à haute fréquence pendant une période de temps et dans lequel l'unité de mesure (120 ; 220 ; 320) est configurée pour acquérir la mesure pendant la période de temps et l'unité de traitement (130 ; 230 ; 330) est configurée pour déterminer la relation de phase pendant la période de temps.

8. Dispositif selon une quelconque des revendications précédentes, dans lequel l'unité de traitement (130 ; 230 ; 330) est configurée pour commander l'unité de génération de stimulation (110 ; 210 ; 310) afin d'adapter au moins un du premier signal de stimulation à haute fréquence ou du deuxième signal de stimulation à haute fréquence, dans le but de modifier la relation de phase entre le signal neuronal et le signal de stimulation interférentielle.

9. Dispositif selon la revendication 1, dans lequel l'unité de génération de stimulation (310) est configurée pour générer un troisième signal de stimulation à haute fréquence et un quatrième signal de stimulation à haute fréquence pour stimuler le cerveau et/ou un nerf de l'être vivant, dans lequel une différence entre une troisième fréquence du troisième signal de stimulation à haute fréquence et une quatrième fréquence du quatrième signal de stimulation à haute fréquence définissent une fréquence de battement ;
dans lequel l'ensemble d'électrodes (302) comprend un premier sous-ensemble d'électrodes (303a) configuré pour recevoir le premier signal de stimulation à haute fréquence et le deuxième signal de stimulation à haute fréquence et configuré pour être disposé à un premier emplacement par rapport au cerveau et/ou au nerf afin de provoquer une stimulation interférentielle par un premier signal de stimulation interférentielle formant ledit signal interférentiel et dans lequel le dispositif (300) comprend en outre un deuxième sous-ensemble d'électrodes (303b) configuré pour recevoir le troisième signal de stimulation à haute fréquence et le quatrième signal de stimulation et configuré pour être disposé à un deuxième emplacement différent du premier emplacement par rapport au cerveau et/ou au nerf afin de transmettre le troisième signal de stimulation à haute fréquence et le quatrième signal de stimulation à haute fréquence dans le cerveau et/ou le nerf, dans lequel le deuxième ensemble d'électrodes (303b) est configuré pour provoquer une stimulation interférentielle dans le cerveau et/ou le nerf par un deuxième signal de stimulation interférentielle ayant la fréquence de battement, dans lequel le deuxième signal de stimulation interférentielle est formé par interférence par le troisième signal de stimulation à haute fréquence et le quatrième signal de stimulation à haute fréquence ;
dans lequel l'unité de traitement (330) est configurée pour déterminer une relation de phase entre le signal neuronal et le premier signal de stimulation interférentielle et dans lequel l'unité de traitement (330) est en outre configurée pour commander le deuxième signal de stimulation interférentielle sur la base de la relation de phase déterminée.

10. Dispositif selon la revendication 9, dans lequel l'unité de traitement (330) est configurée pour commander le deuxième signal de stimulation interférentielle afin de commander le blocage ou le renforcement d'un signal neuronal déclenché par le premier signal de stimulation interférentielle.

11. Dispositif selon une quelconque des revendications précédentes, dans lequel l'ensemble d'électrodes (202 ; 302, 303a, 303b) et l'unité de mesure (220 ; 320) sont disposés dans un ou plusieurs manchons (204 ; 304a, 304b, 304c) configurés pour être disposés autour d'un nerf afin fournir une stimulation du nerf.
